(19) Europäisches Patentamt
European Patent Office
Office européen des brevets

(11) EP 4 324 910 A1

(12) **EUROPEAN PATENT APPLICATION**
published in accordance with Art. 153(4) EPC

(43) Date of publication:
**21.02.2024 Bulletin 2024/08**

(21) Application number: **22936717.2**

(22) Date of filing: **08.11.2022**

(51) International Patent Classification (IPC):
***C12N 1/20** (2006.01)* ***A01G 17/02** (2006.01)*
***A01G 13/00** (2006.01)* ***A01N 63/22** (2020.01)*
***A01P 3/00** (2006.01)* ***A01P 21/00** (2006.01)*
***C05F 11/08** (2006.01)* ***C12R 1/07** (2006.01)*

(52) Cooperative Patent Classification (CPC):
Y02A 40/10

(86) International application number:
**PCT/CN2022/130479**

(87) International publication number:
**WO 2023/240910 (21.12.2023 Gazette 2023/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.06.2022 CN 202210662623**

(71) Applicant: **Beijing Academy of Agriculture and Forestry Sciences**
**Beijing 100097 (CN)**

(72) Inventors:
- **YAN, Jiye**
  **Beijing 100097 (CN)**
- **LI, Xinghong**
  **Beijing 100097 (CN)**
- **ZHANG, Wei**
  **Beijing 100097 (CN)**

(74) Representative: **Grünecker Patent- und Rechtsanwälte PartG mbB**
**Leopoldstraße 4**
**80802 München (DE)**

Remarks:
The complete document including Reference Table(s) and the Sequence Listing(s) can be downloaded from the EPO website

(54) **BACILLUS HALOTOLERANS AND APPLICATION THEREOF**

(57) A strain of *Bacillus halotolerans* and use thereof is provided in the present disclosure. The preservation number of strain BJ-3 of the *Bacillus halotolerans* is CGMCC No. 24115. The *Bacillus halotolerans* has bacteriostatic activity against plant pathogenic fungi and has a broad bacteriostatic spectrum. The results of indoor and field trials indicate that the BJ-3 fermentation broth has certain control efficiency on grapevine trunk diseases. In addition, the fermentation filtrate of the strain significantly promotes the leaf area and the chlorophyll content of grapevines. The *Bacillus halotolerans* has biocontrol potential and can be used to prepare a biocontrol agent and microbial fertilizer against plant pathogenic fungi.

FIG. 1

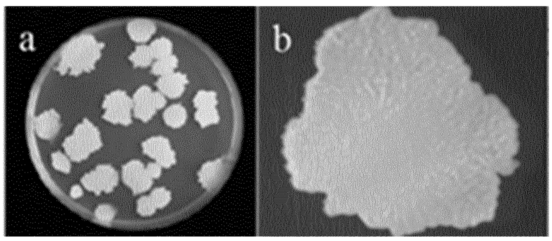

## Description

### TECHNICAL FIELD

The present disclosure belongs to the microbial application field, and in particular, to a strain of *Bacillus halotolerans* and its application for controlling plant diseases such as grapevine trunk diseases (GTDs).

### BACKGROUND ART

Grape (*Vitis vinifera* L.) is one of the important fruit crops in the world. In 2020, the global grape cultivation area is 6,950,900 hectares, and its output is 78,034,300 tons. China's grape cultivation area is 767,500 hectares, ranking second in the world, and its output is 14,843,100 tons, ranking first in the world (FAO, 2020). Grapes are widely cultivated in China. However, most ecological areas in China are sub-suitable for grape cultivation, because the weather is hot and rainy in summers, and grapevines need to be buried to prevent damage during winters in northern China. Therefore, grapevine diseases have always been the main threats to grape production and quality in China. Diseases that occur all year round and need to be controlled include grapevine downy mildew, grapevine powdery mildew, grapevine gray mold, grapevine white rot, grapevine anthracnose and grapevine trunk diseases.

Grapevine trunk diseases include several important vascular bundle diseases that harm grapevines, which are mainly consisting of Esca complex disease, *Botryosphaeria* dieback, *Eutypa* dieback, *Diaporthe* dieback and Black foot disease (Qingtong Ye, Yameng Li, Yueyan Zhou, et al. Occurrence of grapevine trunk diseases caused by fungal pathogens in the domestic and overseas. Journal of Fruit Science, 2021, 38(2):278-292.). Grapevine trunk diseases not only cause the decline of grape production and quality, but also shorten the life of vines and cause more serious losses. According to statistics, the worldwide economic cost for the replacement of dead vines alone is roughly estimated to be in excess of 1.5 billion dollars per year. Grapevine trunk disease is considered to be one of the important diseases affecting the sustainable and healthy development of grape industry in the next 30 years (Hofstetter V, Buyck B, Croll D, et al. What if esca disease of grapevine were not a fungal disease?. Fungal Diversity, 2012, 54(1):51-67.). Grapevine trunk diseases are new diseases discovered in the recent decade. Among them, Botryosphaeria dieback and Diaporthe dieback are the major grapevine trunk diseases (Yan J Y, Xie Y, Zhang W, et al. Species of Botryosphaeriaceae involved in grapevine dieback in China. Fungal Diversity. 2013, 61: 221-236. Manawasinghe I S, Dissanayake A J, Li X, et al. High genetic diversity and species complexity of Diaporthe associated with grapevine dieback in China. Frontiers in Microbiology, 2019, 10: 1936.). Due to the characteristics of latent infection of grapevine trunk disease pathogens and the poor targeting effect of endogenic agents, there is still a lack of effective prevention and control measures in current production. Therefore, it is urgent to explore the effective prevention and control measures of grapevine trunk diseases.

Biological control, which is one of the important measures to prevent and control crop diseases, has the advantages of being pollution-free and less likely to develop resistance, etc. In recent years, it has been highly valued and widely applied in the context of green and high-quality agricultural development. *Bacillus* is rich in resources and is the most widely developed and used biocontrol bacteria. *Bacillus* can produce spores resistant to harsh conditions and reproduce quickly, which is conducive to the production, processing, transportation and storage of preparations. *Bacillus* also has a wide range of bacteriostasis. *Bacillus subtilis*, *Bacillus amyloliquefaciens* and *Bacillus pumilus* have been used to control a variety of grapevine diseases such as grapevine gray mold and downy mildew. It has been found that *Bacillus subtilis* can inhibit the growth of *Lasiodiplodia theobromae*, *Neofusicoccum parvum* and other pathogens that cause *Botryosphaeria* dieback and cause hyphal deformity. Field treatment of grape pruning wound can significantly reduce the incidence of *Botryosphaeria* dieback (Rusin C, Rossicavalcanti F, Lima P, et al. Control of the fungi Lasiodiplodia theobromae, the causal agent of dieback, in cv. syrah grapevines. Acta Scientiarum Agronomy, 2020, 43: e44785.). *Bacillus halotolerans* is a type of bacillus with biocontrol potential, and in recent years, its role in the control of plant diseases has been highly valued. Studies have shown that *Bacillus halotolerans* controls cotton verticillium wilt, and also suppresses grapevine gray mold, tomato gray mold, tomato root-knot nematode, wheat scab, pea root rot and the like (Eirini-Evangelia Thomloudi, and Panagiotis Katinakis. Genomic and metabolomic insights into secondary metabolites of the novel Bacillus halotolerans Hil4, an endophyte with promising antagonistic activity against gray mold and plant growth promoting potential, Microorganisms, 2021, 9: 2508; Muhammad Nadeem Hassan, Vegetable associated Bacillus spp. suppress the pea (*Pisum sativum* L.) root rot caused by Fusarium solani Raheela Riaz 1, Biological Control 2021: 104610; Strain of Bacillus halotolerans LYSX1-induced systemic resistance against the root-knot nematode Meloidogyne javanica in tomato, Ann Microbiol (2019) 69:1227-1233). In addition, *Bacillus halotolerans* also promotes plant growth (Eirini-Evangelia Thomloudi, and Panagiotis Katinakis. Genomic and metabolomic insights into secondary metabolites of the novel Bacillus halotolerans Hil4, an endophyte with promising antagonistic activity against gray mold and plant growth promoting potential, Microorganisms, 2021, 9:2508). Therefore, the screening of *Bacillus* which controls grapevine trunk diseases can open up a new way for the effective control of grapevine trunk diseases. In consideration of the difference of biocontrol potential between different strains of the same *Bacillus* species to some extent, the biocontrol potential of target strains should be deeply explored and fully exploited during the development and application of biocontrol preparations of *Bacillus.*

SUMMARY

To solve the above problem, a strain of *Bacillus halotolerans* and application thereof is provided.

The *Bacillus halotolerans* in the present disclosure, classified and named as *Bacillus halotolerans*, is collected from Huailai County, Zhangjiakou, Hebei Province, isolated from the trunk tissues with grapevine trunk diseases, and named as Bacillus halotolerans BJ-3,which was deposited in the General Microbiology Center of China Committee of Microbiological Culture Collection and Management (Address: No. 3, No. 1 Courtyard, West Beichen Road, Chaoyang District, Beijing) on December 16, 2021, with the preservation number of CGMCC No. 24115.

The *Bacillus halotolerans* of the present disclosure does not produce pigment on LB medium, and colonies are opalescent and opaque, round or nearly round, with non-smooth edges and obvious folds on surface. The physiological and biochemical characteristics of the *Bacillus halotolerans* BJ-3 are Gram staining positive and can produce cellulase, but does not produce protease and amylase.

The *Bacillus halotolerans* has bacteriostatic activity against plant pathogenic fungi and has a broad bacteriostatic spectrum. The plant pathogenic fungi include *Lasiodiplodia theobromae*, *Botryosphaeria dothidea*, *Neofusicoccum parvum*, *Diaporthe sojae*, *Diaporthe eres*, *Diaporthe honkonggensis*, *Botrytis cinerea*, *Colletotrichum viniferum*, *Colletotrichum aenigma*, *Colletotrichum gloeosporioides*, *Colletotrichum fructicola*, *Colletotrichum siamense*, *Colletotrichum acutatum*, *Neopestalotiopsis sp.*, *Neopestalotiopsis rosae*, *Coniella vitis*, *Dactylonectria macrodidyma*, *Fusarium oxysporum*, anastomosis group A of *binucleate Rhizoctonia*, anastomosis group G of *binucleate Rhizoctonia*, *Alternaria alternata*, and *Sclerotinia sclerotiorum.* Inhibition rates of mycelial growth of 45 strains of the above 22 plant pathogenic fungi are between 54.74% and 91.62%. These strains have a very good application prospect in the development of biological agents for plant fungal diseases.

The *Bacillus halotolerans* BJ-3 strain in the present disclosure can inhibit the conidial germination of Lasiodiplodia theobromae with an $EC_{50}$ value of $3.35\times10^6$ cfu/mL.

The results of indoor in vitro branch inoculation method for disease prevention and field efficacy trials indicate that the *Bacillus halotolerans* BJ-3 strain in the present disclosure controls grapevine *Botryosphaeria* dieback well. The *Bacillus halotolerans* BJ-3 strain in the present disclosure obviously inhibits *Botryosphaeria* dieback on an in vitro green shoot, and the protective effect and treatment effect of strain BJ-3 fermentation broth at a concentration of $1.08\times10^8$CFU/mL on the control of Botryosphaeria dieback are 91.75% and 86.94%, respectively.

If the concentration of the fermentation broth of the *Bacillus halotolerans* BJ-3 strain is $5.6\times10^7$ CFU/mL, the control efficiency on grapevine trunk diseases in the field could reach 65.56%, which is significantly better than that of biocontrol agent and chemical agent as control, and have a biocontrol potential to control Botryosphaeria dieback. The *Bacillus halotolerans* BJ-3 strain can be used to prepare biocontrol agent or microbial fertilizer for controlling grapevine trunk disease fungi. The pathogenic fungi of grapevine trunk diseases include *Lasiodiplodia theobromae*, *Neofusicoccum parvum*, *Botryosphaeria dothidea*, *Diaporthe eres*, *Diaporthe sojae*, *Neopestalotiopsis sp.*, and *Dactylonectria macrodidyma.*

Application of the *Bacillus halotolerans* in promoting growth of grape seedlings also falls within the protection scope of the disclosure. In the application, roots of the grape seedlings are irrigated with fermentation medium, diluent of the fermentation medium, fermentation medium filtrate or diluent of fermentation medium filtrate of the *Bacillus halotolerans.*

Preferably, when the grape seedlings grow to have 5-7 leaves, the roots are irrigated with diluent of the fermentation medium filtrate. In a preferred technical solution of the present disclosure, the diluent of the fermentation culture filtrate is obtained by diluting the fermentation culture filtrate 10,000 times with water.

The diluent of the fermentation medium filtrate is obtained by the following steps: pouring seed solution into a 500 mL triangular flask containing 100 mL fermentation medium (maltose 20 g/L, peptone 10 g/L, bran 10 g/L, $CaCO_3$ 10 g/L, $NaHPO_3$ 2 g/L, $KH_2PO_3$ 1 g/L; pH value 6.8) with 10% of inoculation amount, and culturing at 28 °C for 72 hours with shaking at 180 rpm/min to obtain fermentation broth; centrifuging the fermentation broth by a high-speed refrigerated centrifuge at 4 °C at 10,000 rpm for 15-20 minutes, collecting and filtering supernatant through a bacterial filter, and discarding precipitation to obtain sterile fermentation filtrate.

The *Bacillus halotolerans* has bacteriostatic activity against plant pathogenic fungi and has a wide bacteriostatic spectrum, especially for controlling Botryosphaeria dieback. The strain promotes growth of grape seedlings well. The strain needs simple culture condition, grows rapidly and is easily preserved. Therefore, it is suitable for industrial production.

## BRIEF DESCRIPTION OF THE DRAWINGS

FIG. 1 shows colony morphology and SEM photos of strain BJ-3.

FIG. 2 shows results of amylase detection of strain BJ-3, wherein the left is *Escherichia coli* JM109, and the right is strain BJ-3.

FIG. 3 shows results of protease detection of strain BJ-3, wherein the left is *Escherichia coli* JM109, and the right is strain BJ-3.

FIG. 4 shows results of cellulase detection of strain BJ-3, wherein the left is *Escherichia coli* JM109, and the right is strain BJ-3.

FIG. 5 shows a phylogenetic tree based on 16SrDNA.

FIG. 6 shows a phylogenetic tree based on gyrB gene.

FIG. 7 shows growth-promoting effects of different treatments of strain BJ-3 on grape seedlings (42nd day), wherein a is CK; b is 10,000 times diluent of fermentation medium for BJ-3 culture; c is 10,000 times diluent of BJ-3 fermentation filtrate; d is $5.8\times10^6$ CFU/mL BJ-3 fermentation bacterial broth.

FIG. 8 shows effects of different treatments of strain BJ-3 on shoot biomass of grape seedlings, wherein treatment 1 is BJ-3 fermentation broth at a concentration of $5.8\times10^6$ CFU/mL, treatment 2 is 10,000 times diluent of fermentation medium for BJ-3 culture, treatment 3 is 10,000 times diluent of BJ-3 fermentation filtrate, and treatment 4 is clean water control.

FIG. 9 shows effects of different treatments of strain BJ-3 on leaf area of grape seedlings, wherein treatment 1 is BJ-3 fermentation broth at a concentration of $5.8\times10^6$ CFU/mL, treatment 2 is 10,000 times diluent of BJ-3 fermentation medium, treatment 3 is 10,000 times diluent of BJ-3 fermentation filtrate, and treatment 4 is clean water control.

FIG. 10 shows effects of different treatments of strain BJ-3 on chlorophyll content of grape seedlings, wherein treatment 1 is BJ-3 fermentation broth at a concentration of $5.8\times10^6$ CFU/mL, treatment 2 is 10,000 times diluent of BJ-3 fermentation medium, treatment 3 is 10,000 times diluent of BJ-3 fermentation filtrate, and treatment 4 is clean water control.

## DETAILED DESCRIPTION

Example 1. Acquisition and identification of strain BJ-3

The pathogens are isolated by a tissue separation method from grapevine trunk tissues showing symptoms of grapevine trunk diseases, collected from Huailai County, Zhangjiakou, Hebei Province. The method includes: (1) observing and record samples showing symptoms of grapevine trunk diseases; (2) for the parts with different severity of disease, cut and collect 2 cm of grapevine branches every 10 cm, removing bark of the samples showing symptoms of grapevine trunk diseases, cutting and collecting 5 mm$^2$ tissue blocks from the disease and health boundary of the branches, disinfect the tissue blocks with 2% sodium hypochlorite for 2 minutes, 70% ethanol for 30 seconds, and wash with sterile water for 3 times, and putting sterilized filter paper on the tissue blocks to dry; after the moisture is sucked up, placing the tissue blocks on PDA plates, where 4 to 5 tissue blocks are placed on each PDA plate, and seal with parafilm ; (3) culturing at 25°C under dark conditions for 3 days, and after 3 days observation, record colony growth and calculating corresponding segregation ratios; (4) pick a small amount of mycelia at the edge of the colony in a new PDA or other medium that is easy to induce sporulation, and isolating single spores after conidia are produced. In the process of isolating pathogenic fungi, a strain of bacteria is found to have a good antagonistic effect on pathogenic fungi. The strain of bacteria is striated and purified on a LB medium plate to obtain a pure culture of bacteria. After identification, a strain with antagonistic effect against pathogens are obtained and named BJ-3. The colony morphology of strain BJ-3 is shown in FIG. 1. The colony is opalescent and opaque, round or nearly round, with non-smooth edges and obvious folds on surface.

1. Physiological and biochemical characteristics of strain BJ-3

1.1 Amylase Detection

Detection medium: Amylase selected medium (1L): 1g soluble starch, 5g peptone, 5g glucose, 5g NaCl, 5g beef extract, and 15g agar.

Detection method: after 3 days of culture of a to-be-detected strain in LB solid medium, picking a single colony site and seeding it to the Amylase detection medium, culturing at a constant temperature of 28 °C for 24-48 hours, and observing the transparent circle.

Detection result: as shown in FIG. 2, there is no obvious transparent circle around the colony of the to-be-detected strain, indicating that the strain BJ-3 does not produce Amylase.

1.2 Protease detection

Detection plate: protease selected medium (1 L): Peptone 10.0 g, NaCl 5.0 g, $CaCl_2$ 0.1 g, skimmed milk 100.0 mL (added at 45 °C after sterilization), and agar 15 g.
Detection method: after culturing the to-be-detected strain in the LB solid medium for 3 days, picking a single colony site and seeding it on the protease detection medium, culturing at a constant temperature of 28 °C for 24-48 hours, and observing the transparent circle.
Detection result: as shown in FIG. 3, there is no obvious transparent circle around the colony of the to-be-detected strain, indicating that the strain BJ-3 does not produce protease.

1.3 Cellulase detection

Detection plate: Cellulase selected medium (1 L): CMC-Na 10 g, peptone 10 g, yeast powder 10 g, $KH_2PO_4$ 1 g, NaCl 15 g, and agar 15 g.
Detection method: after culturing the to-be-detected strain in LB solid medium for 3 days, pick a single colony site and seeding it to the cellulase detection medium, culturing at a constant temperature of 28 °C for 24-48 hours, and observing the transparent circle.
Detection method: as shown in FIG. 4, there is an obvious transparent circle around the colony of the to-be-detected strain, indicating that strain BJ-3 produces cellulase.

2. Molecular identification of strain BJ-3

2.1 Extraction of Genomic DNA:

After plating the preserved bacterial solution on a petri dish and culturing at 25 °C for 3 days, a bacterial body is scraped off with an inoculation loop and washed out using LB liquid medium. The bacterial genomic DNA is extracted using a rapid bacterial genomic DNA isolation kit of Beijing Biomed Gene Technology Co., Ltd.

2.2 PCR amplification of 16S rDNA:

The genomic DNA of BJ-3 is extracted, and the genomic DNA of this bacterial strain is taken as a template for PCR amplification using 16S rDNA universal primers 27f and 1492r.
Primers used for PCR amplification:

27f: 5'-AGAGTTTGATCCTGGCTCAG-3'

1492r: 5'-TACCTTGTTACGACTT-3'.

Reaction system for PCR amplification: 2 × Taq PCR Mix 25 μL, DNA template (100 ng/μL) 2 μL, each of upstream and downstream primers (10 μmol/L) 1 μL, 50 μL system supplemented with sterile dd$H_2O$.
PCR reaction procedure: 94 °C for 3 minutes; 94 °C for 30 seconds, 54 °C for 30 seconds, 72 °C for 90 seconds, 35 cycles; 72 °C for 10 minutes; heat preservation: 4 °C.
The PCR amplification product is detected by electrophoresis, with a fragment length of approximately 1500 bp (SEQ NO: 1 in the sequence listing).

2.3 PCR amplification of gyrB gene

The genomic DNA of bacteria BJ-3 is extracted, and the genomic DNA of this strain is taken as a template for PCR amplification using the universal primers gyrB-F and gyrB-R for bacterial gyrB genes.
Primers used for PCR amplification:

gyrB-F: 5'-TTGRCGGHRGYGGHTATAAAGT-3'
gyrB-R: 5'-TCCDCCSTCAGARTCWCCCTC-3'

Reaction system for PCR amplification: 2 × Taq PCR Mix 25 μL, DNA template (100 ng/μL) 2 μL, each of

upstream and downstream primers (10 μmol/L) 1 μL, 50 μL system supplemented with sterile $ddH_2O$.

PCR reaction procedure: 94 °C for 4 minutes; 94 °C for 30 seconds, 55 °C for 45 seconds, 72 °C for 1 minute, 35 cycles; 72 °C for 10 minutes; heat preservation: 4 °C.

The PCR amplification product is detected by electrophoresis, with a fragment length of approximately 900 bp (SEQ NO:2 in the sequence listing).

2.4 Sequence Analysis and Molecular Identification

The PCR product is verified by 1% agarose gel electrophoresis and sent to Beijing Biomed Gene Technology Co., Ltd. for sequencing. The obtained sequence is compared with the sequence in the GenBank database of the NCBI website, and the strain type with the highest similarity to the 16S rDNA sequence and gyrA gene sequence is obtained. The results indicate that the 16S rDNA sequence similarity between the BJ-3 strain and the strain of *Bacillus halotolerans* TRM85649 is 98.47%, and the gyrB gene sequence similarity between the BJ-3 strain and the strain of *Bacillus halotolerans* LYSX1 is 99.77%. The BJ-3 strain is preliminarily identified as a closely related population strain of *Bacillus halotolerans.*

The sequencing results of 16S rDNA and gyrB gene PCR amplified fragments of strain BJ-3 are shown in SEQ NO:1 and SEQ NO:2 in the sequence listing, respectively.

Based on the gene fragment sequence and using MEGA software, phylogenetic trees are constructed using ML method based on 16S rDNA and gyrB genes, as shown in FIGs. 5 and 6. The results indicate that strain BJ-3 is aggregated with strain of *Bacillus halotolerans* CR-119 (GenBank registration number: NR115283) and strain of *Bacillus halotolerans* CECT 5687 (GenBank registration number: DQ903179), respectively. Based on this, the strain BJ-3 is identified as *Bacillus halotolerans.* The strain BJ-3 is deposited in the China General Microbiological Culture Collection Center (Address: Institute of Microbiology, Chinese Academy of Sciences, No. 3, No. 1 Courtyard, West Beichen Road, Chaoyang District, Beijing) on December 16, 2021, with the presevation number of CGMCC No. 24115.

Example 2. Determination of Bacteriostatic activity of Strain BJ-3 CGMCC No. 24115

The antagonistic activity of strain BJ-3 is determined by the confrontation culture method, taking 45 strains of 22 plant pathogenic fungi (the information of plant pathogenic fungal strains is shown in Table 1, the plant pathogenic fungal strains are identified by the Plant Disease Control Research Office of Beijing Academy of Agriculture and Forestry Sciences according to morphological and molecular identification methods and Koch's rule) as target bacteria.

BJ-3 is cultured on LB solid medium at 25 °C for 2-3 days, a single colony is picked and placed in 1 mL of LB liquid medium, shaken at 37 °C at 200 rpm/min for 8-10 hours for later use. 45 pathogen strains are cultured on PDA plates for 3-5 days. A pathogen cake with a diameter of 5 mm is made by drilling holes in the edge area of the colony with a hole punch and placed in the center of the PDA plate. Simultaneously, 4μL of cultured bacterial suspension are absorbed and dropwise added on a filter paper sheet with a diameter of 6mm, and placed about 1cm away from the edge of the plate. 4 filter paper sheets are placed on each plate, and the same dose of water was added to the filter paper sheets as control in triplicate, and cultured in an incubator at 28 °C for 5 days to observe the generation of bacteriostatic zone, measure the radius (D) and width of the bacteriostatic zone (d) in the direction of pathogen mycelia growth relative to the endogenous antagonistic bacteria BJ-3, and calculate the inhibition rate.

$$\text{Inhibition rate (\%)} = (\text{a control colony radius - a treated colony radius})/\text{a control colony radius} \times 100\%$$

Table 1. Bacteriostatic activity of strain BJ-3 against 45 plant pathogenic fungi

| Disease name | Pathogen | Strain number | Inhibition rate (%) |
|---|---|---|---|
| Grapevine botryosphaeria dieback | *Botryosphaeria dothidea* | BD-3 | 71.26 |
| | | GXZY-02S | 76.18 |
| | *Lasiodiplodia theobromae* | CSS-01S | 69.89 |
| | | GX-5-5 | 55.56 |
| | *Neofusicoccum parvum* | AH-3-1-01S | 65.81 |
| | | GX-1 | 70.59 |
| Grapevine diaporthe dieback | *Diaporthe sojae* | GG-6 | 74.18 |
| | | OSS① | 91.62 |
| | *Diaporthe eres* | HS3-1 | 64.54 |
| | | DB-3 | 81.31 |
| Grapevine gray mold | *Botrytis cinerea* | SX-gray | 62.64 |
| | | HB-gray (1) | 63.72 |
| | | HB-gray (2) | 61.54 |
| Grapevine anthracnose | *Colletotrichum viniferum* | Yt-gray | 63.96 |
| | | BJ-2-gray | 69.57 |
| Grapevine twig blight | *Neopestalotiopsis sp.* | JL-4-⑤ | 77.78 |
| Grapevine white rot | *Coniella vitis* | JZB3700021 | 56.59 |
| | | JZB3700014 | 59.27 |
| Grapevine black foot disease | *Dactylonectria macrodidyma* | JZB3310008 | 74.08 |
| Strawberry root rot | *Neopestalotiopsis rosae* | JZB340066 | 60.98 |
| | | JZB340065 | 70.64 |
| | *Fusarium oxysporum* | LGS15 | 62.77 |
| | *Colletotrichum siamense* | JZB330161 | 57.50 |
| | *Anastomosis group A of binucleate Rhizoctonia* | JZB333001 | 68.38 |
| | | JZB333002 | 68.38 |
| | *Anastomosis group G of binucleate Rhizoctonia* | JZB333003 | 64.96 |
| Cherry Chaetospora leaf spot | *Alternaria alternata* | A1 | 64.13 |
| | | A2 | 54.74 |
| Cherry anthracnose leaf spot | *Colletotrichum aeniggma* | JZB330210 | 68.18 |
| | | JZB330211 | 66.35 |
| | *Colletotrichum gloeosporioides* | JZB330237 | 64.91 |
| | | JZB330239 | 72.57 |
| | *Colletotrichum fructicola* | JZB330259 | 55.38 |
| | | JZB330261 | 56.04 |

(continued)

| Disease name | Pathogen | Strain number | Inhibition rate ( % ) |
|---|---|---|---|
| Other cherry leaf spot | *Botryosphaeria dothidea* | JZB310198 | 70.94 |
| | | JZB310202 | 67.52 |
| | *Diaporthe sojae* | JZB320189 | 65.22 |
| Cherry trunk disease | *Botryosphaeria dothidea* | GZZG7-A-S1 | 67.52 |
| | | SYZG-13-A-S1 | 65.81 |
| | *Diaporthe honkonggensis* | GZZG2-S1 | 60.00 |
| | | GZZG2-S3 | 63.25 |
| | *Diaporthe eres* | TZZG36-S1 | 69.23 |
| Leek sclerotinia disease | *Sclerotinia sclerotiorum* | BJTZ-SS-1 | 58.91 |
| Pepper anthracnose | *Colletotrichum acutatum* | BJYH-CG-3 | 59.69 |
| Eggplant gray mold | *Botrytis cinerea* | BJTZ-BC-1 | 56.60 |

The bacteriostatic activity of strain BJ-3 against 45 strains of 22 plant pathogenic fungi is obtained using the confrontation culture method, and the result is shown in Table 1. It can be seen from the experimental result that strain BJ-3 has a significant inhibition effect on different test plant pathogenic fungi, with an inhibition rate of mycelial growth between 54.74% and 91.62%. Strain BJ-3 has the best inhibition effect on the mycelial growth of Diaporthe sojae OSS① that causes Diaporthe dieback, with an inhibition rate of 91.62%. Research results indicate that BJ-3 has a broad-spectrum inhibition effect on common plant pathogens.

**Example 3. Determination of the inhibition effect of strain BJ-3 on the conidial germination of Lasiodiplodia theobromae of Grapevine Botryosphaeria dieback**

1. Experimental method

1.1 Preparation of conidial suspension of Lasiodiplodia theobromae

The preserved Lasiodiplodia theobromae strain GX-5-5 of Grapevine Botryosphaeria dieback is inoculated on a PDA plate and cultured at 28°C for 2-3 days. After that, the activated bacteria are transferred to a new PDA plate and cultured at 28 °C for 7~10 days. After sporulation, the conidia are eluted with sterile water and filtered with 4 layers of sterile gauze to remove the mycelia and impurities. Finally, the conidial suspension is diluted with sterile water to $1\times10^5$ spores /mL for later use.

1.2 Preparation of bacterial suspension of strain BJ-3

The strain BJ-3 is cultured on LB solid medium at 28 °C for 2-3 days, and a single colony is picked and placed in 20 mL of LB liquid medium, shaken at 28 °C at 200 rpm/min for 8-10 hours for later use.

1.3 Determination of inhibition effect of BJ-3 strain suspension on spore germination of Botryosphaeria dieback

The concentration of BJ-3 bacterial suspension prepared is adjusted with sterile water to $8\times10^5$, $1.6\times10^6$, $4\times10^6$, $8\times10^6$, $2\times10^7$, and $4\times10^7$ CFU/mL, and the spore suspension is mixed with 6 kinds of bacterial suspensions at different concentrations of BJ-3 at volume ratio of 1:1. The spore suspension and sterile water at volume ratio of 1:1 is used as control. Each treatment is repeated 3 times. The mixed liquor of 100 $\mu$L is absorbed and dropwise added to a sterilized concave slide. The concave slide is placed in a petri dish covered with a wet filter paper and cultured at 28°C in an incubator. The conidial germination is observed by a microscope at 10-12 h.

1.4 Data statistics and analysis

The spore is regarded as germinated spore when the length of a conidial tube exceeds 1/2 of the diameter of the spore. When the germination rate of a blank control spore reaches more than 90%, the germination of each treatment is checked. More than 3 views are randomly observed in each replicate of each treatment, and the total number of spores investigated is not less than 200. The germination number and total number of spores are recorded respectively. Then, the virulence regression equation, correlation coefficient (r) and $EC_{50}$ are obtained by converting the bacterial concentration-inhibition rate into a corresponding log-probability value.

Calculation formula:

$$R=Ng / Nt \times 100$$

(R-spore gemination rate; Ng-spore gemination number ; Nt-total number of spores investigated ) ,

$$Re=Rt / R_0 \times 100$$

(Re-treated and adjusted spore germination rate; Rt -treated spore germination rate; $R_0$-gemination rate of blank control spore),

$$I=(R_0-Re)/ R_0 \times 100 \text{ (I-relative inhibition rate of spore germination).}$$

2. Experimental results

Indoor biological activity tested results of strain BJ-3 against Lasiodiplodia theobromae GX-5-5 are shown in Table 2. The results indicate that BJ-3 bacterial solution has obvious inhibition effect on spore germination of GX-5-5. The inhibition rate of BJ-3 bacterial solution at a test concentration of $8\times10^5$~$4\times10^7$ CFU/mL against conidia germination of Lasiodiplodia theobromae is 16.72%-98.47%. When the concentration of BJ-3 bacterial suspension is $2\times10^7$ CFU/mL and $4\times10^7$ CFU/mL, the inhibition rates of spore germination are 97.95% and 98.47% respectively, the $EC_{50}$ value is $3.35\times10^6$ cfu/mL, and the r value is above 0.9. Therefore, the strain BJ-3 has a strong inhibition effect on spore germination of Lasiodiplodia theobromae, which may have the ability to control Botryosphaeria dieback by inhibiting the infection of bacteria to host.

Table 2.Indoor biological activity tested results of strain BJ-3 against Lasiodiplodia theobromae GX-5-5

| Treatment concentration ( CFU/mL ) | Corrected inhibition rate (%) | Linear regression equation | r value | $EC_{50}$ value |
|---|---|---|---|---|
| $8\times10^5$ | 16.72 | y = 2.0253x - 8.2145 | 0.9579 | $3.35\times10^6$ |
| $1.6\times10^6$ | 29.23 | | | |
| $4\times10^6$ | 31.54 | | | |
| $8\times10^6$ | 71.02 | | | |
| $2\times10^7$ | 97.95 | | | |
| $4\times10^7$ | 98.47 | | | |

## Example 4. Determination of control efficiency of BJ-3 strain on Botryosphaeria dieback by in vitro branch inoculation

1. Experimental method

1.1 Preparation of fermentation broth of strain BJ-3

A single colony of strain BJ-3 is picked with a sterilized toothpick and inoculated in 10 mL LB liquid medium, cultured with shaking at 28°Cat 180 rpm for 12 hours, and then transferred to a triangle flask containing 100 mL fermentation medium, cultured with shaking at 28°Cat 180 rpm for 72 hours to obtain fermentation broth of strain BJ-3. The fermentation broth is diluted with sterile water to produce fermentation broths at three concentrations of $10^6$ CFU/mL, $10^7$ CFU/mL and $10^8$ CFU/mL and stored at 4°C.

1.2 Culture and preparation of inoculant and test pathogens

A green shoot of annual grape is selected as an inoculant. A green shoot of current-year and semi-lignified Vitis Vinifera 'Summer Black' is collected from the vineyard on the day of inoculation. After cutting the leaves, the surface is cleaned with water, then wiped and disinfected with 75% alcohol wipes, and the surface is rinsed with sterile water to dry for later use.

The test pathogen is strain GX-5-5 of Lasiodiplodia theobromae, and the test pathogen is cultured on PDA medium at 28°C for 3 days and then reserved.

1.3 Control efficiency of strain BJ-3 on Botryosphaeria dieback

1.3.1 Determination of the protective effect of strain BJ-3 on Botryosphaeria dieback

The green shoot of disinfected grape is treated with the fermentation broth of strain BJ-3 by spraying, and the treatment concentrations are BJ-3 $1.08\times10^6$ CFU/mL, $1.08\times10^7$ CFU/mL, and $1.08\times10^8$ CFU/mL, respectively. Then the green shoot is inserted into a plastic bowl having a diameter of 9 cm and containing wet vermiculite substrate, and cultured in an artificial climate chamber at 25°C and 60% relative humidity for 24 hours. Subsequently, a wound with a diameter of 4 mm and a depth of 1 mm is made in the middle of the internode of the shoot by a sterilized hole punch with a diameter of 4.0 mm. The tissues of the wound are peeled off. The fungal cake that is made with 4.0 mm-hole punch at 1 cm inward along the colony edge of Lasiodiplodia theobromae cultured for 3 days is placed on the wound of the branch with a sterilized toothpick, and the mycelium surface contacts the grape tissue. The location of the inoculated fungal cake is wrapped with a parafilm, and then reinserted into the plastic bowl. A green shoot inoculated with blank PDA fungal block and sprayed with endogenic antagonistic bacteria fermentation broth, a green shoot inoculated with Lasiodiplodia theobromae and sprayed with sterile water, a green shoot inoculated with a blank PDAfungal block and sprayed with sterile water are used as controls, and $5\times10^6$ CFU/ml, 10 billion CFU/mL Bacillus subtilis WP are used as control agents. For each treatment, 7 shoots is inoculated, in triplicate. After inoculation, the culture is continued in the inoculation room at 25°C with 90% relative humidity for 48 hours. After that, the relative humidity is adjusted to 60%. The incidence and the lesion length of each treatment are measured after 7-10 days of inoculation, and the data is statistically analyzed by software SPSS 18. The calculation formula is as follows:

$$\text{Control efficiency (\%)} = [(\text{a length of control spot - a length of treated spot})/(\text{a length of control spot -a diameter of fungal cake})]\times100\%$$

1.3.2 Determination of the therapeutic effect of strain BJ-3 on Botryosphaeria dieback

Determination of the therapeutic effect includes: inoculating the pathogens first, and treating the inoculated green shoots with different bacterial solution and sterile water for 48 hours after inoculation. The method and materials used are the same as those used to determine the protective effect.

2. Experimental result

The control efficiency of strain BJ-3 on Botryosphaeria dieback is obtained through artificial inoculation of in vitro green shoot, and the results are shown in Table 3. The results indicate that there is no significant difference between strain BJ-3 fermentation broths at the three concentrations and $5\times10^6$ CFU/ml,10 billion CFU/mL Bacillus subtilis WP in controlling Botryosphaeria dieback, among which $1.08\times10^8$ CFU/mL BJ-3 fermentation broth has the best control efficiency (91.75%). The therapeutic effect of $1.08\times10^8$ CFU/mL BJ-3 fermentation broth against Botryosphaeria dieback is significantly higher than that of BJ-3 fermentation broth at other concentration and the control agent Bacillus, and the control efficiency is 86.94%; the control efficiency of the control agent Bacillus is 73.72%; and the control efficiency of BJ-3 fermentation broth at a concentration of $1.08\times10^7$ CFU/mL on Botryosphaeria dieback is 51.03%. These control efficiency are not significantly different from that of the control agent. It can be seen that the therapeutic effect of strain BJ-3 on Botryosphaeria dieback is better than that of control agent Bacillus subtilis.

Table 3.Control efficiency of BJ-3 fermentation broth on Botryosphaeria dieback (indoor artificial inoculation)

| Treatment | Protective effect | | Therapeutic effect | |
|---|---|---|---|---|
| | Lesion length (cm) | Control efficiency (%) | Lesion length ( cm ) | Control efficiency (%) |
| BJ-3 1.08×$10^8$ CFU/mL | 1.00±0.10 | 91.75Aa | 1.61±0.05 | 86.94Aa |
| BJ-3 1.08×$10^7$ CFU/mL | 1.37±0.11 | 84.92Aab | 4.42±1.10 | 51.03Bcb |
| BJ-3 1.08×$10^6$ CFU/mL | 1.46±0.00 | 85. 17Aab | 7.93±1.30 | 36.07Dd |
| 10 billion CFU/mL Bacillus subtilis WP 5×$10^6$ CFU/mL | 1.50±0.11 | 86.75Aab | 4.83±0.35 | 73.72Cbc |
| Sterile water | 8.05±2.67 | - | 12.20±1.43 | - |

**Example 5**. **Evaluation of field control efficiency of BJ-3 fermentation broth on grapevine trunk diseases**

1. Experimental materials and method

1. Preparation of fermentation broth of strain BJ-3

Fermentation medium: maltose 20 g/L; Peptone 10 g/L; $CaCO_3$ 10 g/L; $NaHPO_3$ 2 g/L; $KH_2PO_3$ 1g/L, bran 10 g/L; and the balance of water; pH 6.8.

Fermentation conditions: temperature of 32 °C, stirring speed of 200 r/min, ventilation of 1:0.5-1:1.2, culture time of 22 hours. The fermentation broth of BJ-3 is cultured in a 120 L fermenter and obtained at the concentration of 5.6×$10^9$ CFU/mL.

2. Field control of Botryosphaeria dieback by BJ-3 fermentation bacterial broth

The effect of strain BC-3 fermentation broth on the control of grapevine trunk diseases is evaluated by field plot comparison experiment. The experimental object is a grape variety that suffers grapevine trunk diseases all year round and is easily infected. The experimental site is the wine-grape planting base of COFCO Chateau SunGod GreatWall (Huailai), Zhangjiakou City, Hebei Province, and the variety is 'Marselan'. The experiment includes 4 treatments. Treatment 1: BJ-3 fermentation broth at the concentration of 5.6×$10^7$ CFU/mL, treatment 2: 10 billion CFU/g Bacillus subtilis WP 1000 times solution, treatment 3: 10% difenoconazole WG 1000 times solution; and treatment 4: clean water as blank control. The administration method is root irrigation, with 1 L per plant, in triplicate, for each treatment of more than 200 vines. The administration is performed once on July 9, 2021 and once on July 20, respectively. The incidence of vines is investigated before administration and 70 days after administration (close to maturity of grapes), and the disease rate (%) and control efficiency (%) are calculated. The calculation formula is as follows:

A rate of diseased plant (%) = a number of diseased plants/a total number of investigated plants ×100%

Control efficiency (%) = [1- (a rate of diseased plants before treatment in blank control area × a rate of diseased plants after treatment in treated area)/(a rate of diseased plants after treatment in blank control area × a rate of diseased plants before treatment in treated area)]×100%

2. Experimental result

The field disease prevention effect of strain BJ-3 fermentation broth on grapevine trunk diseases indicates (Table 4) that the average control efficiency of 5.6×$10^7$ CFU/mL BC-3 fermentation broth is 65.56%, the average control efficiency of 10 billion CFU/g Bacillus subtilis WP 1000 times solution is 42.34%, and the average control efficiency of 10% difenoconazole WG 1000 times solution is 27.30%. Among them, the effect of BJ-3 fermentation broth at a concentration of 5.6×$10^7$ CFU/mL on the control of Botryosphaeria dieback is significantly higher than that of the other two agent treatments, and there is no significant difference between the latter two agent treatments. In the whole course of the experiment, no harm is found to the test grapes with three kinds of agents.

In summary, the result of the field efficacy test shows that the average efficacy of the two test fungicides and BJ-3 fermentation bacterial broth on the control of grapevine trunk diseases is from 27.30% to 65.56%, among which BJ-3 fermentation bacterial broth (5.6×$10^7$ CFU/mL) has maximum efficacy of 65.56%, which is significant higher than those of Bacillus subtilis and chemical fungicide difenoconazole, having biocontrol potential for controlling grapevine trunk diseases.

Table 4. Field control efficiency of BJ-3 fermentation broth on grapevine diseases

| Agent treatment | Average rate of diseased plants before treatment (%) | Average rate of diseased plants after treatment (%) | Control efficiency (%) |
|---|---|---|---|
| BJ-3 fermentation broth 100 solution ( 5.6×$10^7$ CFU/mL ) | 33.33 | 28.32 | 65.56±2.62aA |
| 10 billion CFU/g Bacillus subtilis WP 1000 times solution | 32.67 | 41.62 | 42.34±2.37bB |
| 10 % Difenoconazole WG 1000 times solution | 18.33 | 32.84 | 27.30±3.24cC |
| CK | 19.0 | 46.95 | - |
| Note: The average rate of diseased plants in the table is the average of three replicates. | | | |

**Example 6. Determination of the growth-promoting effect of strain BJ-3 fermentation broth on grape seedlings**

1. Materials and method

1.1 Preparation of fermentation broth and fermentation filtrate of strain BJ-3

1.1.1 Preparation of seed solution: filling 100 mL sterilized LB liquid medium in a 250 mL triangular flask, and picking a single colony of BJ-3 and transferring it into LB liquid medium, and culturing with shaking at 28 °C at 180 rpm/min for 12 hours.

1.1.2 Fermentation culture: BJ-3: Pouring the seed solution into a 500 mL triangular flask filled with 100mLof fermentation culture medium (Maltose 20 g/L, peptone 10 g/L, bran 10 g/L, $CaCO_3$ 10 g/L, $NaHPO_3$ 2 g/L, $KH_2PO_3$ 1 g/L; pH 6.8) with 10% of inoculation amount, and culturing with shaking at 28 °C at 180 rpm/min for 72 hours to obtain the fermentation broth.

1.1.3 Preparation of fermentation filtrate: centrifuging the fermentation broth at 4 °C at 10,000 rpm for 15-20 minutes by a high-speed refrigerated centrifuge, collecting and filtering the supernatant by a bacterial filter, and discarding the precipitatison to obtain the sterile fermentation filtrate, which is stored at -20 °C for later use.

1.2 Test plant
The test grape variety is "Marselan", and the materials are cuttings. The cuttings are planted in a pot with a diameter of 22.5cm and a height of 13.5cm.

1.3 Growth-promoting effect of strain BJ-3 fermentation broth on grape seedlings
The growth-promoting effect of strain BJ-3 fermentation broth on grape seedlings was determined by root-irrigating. When the grape seedlings grow to have 5-7 leaves, they are root irrigated. Each pot of grape seedlings is root irrigated with 150 mL strain BJ-3 fermentation broth. A total of 4 treatments are set up. Treatment 1: BJ-3 fermentation broth at a concentration of 5.8×$10^6$ CFU/mL; treatment 2: 10,000 times diluent of BJ-3 fermentation medium used for culture (the medium without BJ-3 culture); treatment 3: 10,000 times diluent of BJ-3 fermentation filtrate; and treatment 4: clean water control. Each treatment is used for 10 grape seedlings. The vines are cultured in a greenhouse at 25±2°C.

1.4 Data Statistics and Analysis

On the 7th day, 14th day, 21st day, 28th day, 35th day, and 42th day of root irrigation, the shoot biomass of grape is observed and measured. On the 42nd day, chlorophyll content and leaf area are measured simultaneously, and the data is analyzed using SPSS 25.

2. Results

The experimental results of different treatments of strain BJ-3 on the growth-promotion of grape seedlings are shown in FIGs. 7-10, and the results indicate that the BJ-3 fermentation broth (treatment 1) at the concentration of 5.8 $\times 10^6$ CFU/mL does not significantly promote the shoot biomass of grape seedlings. The 10,000 times diluent of BJ-3 fermentation filtrate (treatment 3) significantly increases the leaf area and chlorophyll content of grape seedlings compared with other treatments, and increases by 8.67% and 8.97% respectively compared with the water control (treatment 4). This indicates that strain BJ-3 may have the ability to improve the photosynthetic performance of grape seedlings by increasing the leaf area and chlorophyll content.

The above examples show only several embodiments of the present disclosure and are described in a specific manner. However, they are only illustrative and not restrictive for the purposes of the disclosure. An ordinary person skilled in the art may make various modifications, variations, and improvements without deviating from the spirit and scope of the attached claims, and these fall within the scope of the protection of this disclosure.

**Claims**

1. A strain of *Bacillus halotolerans*, named as *Bacillus halotolerans* BJ-3, deposited in the General Microbiology Center of China Committee of Microbiological Culture Collection and Management with a preservation number of CGMCC No. 24115.

2. The strain of *Bacillus halotolerans* of claim 1, wherein the *Bacillus halotolerans* has bacteriostatic activity against plant pathogenic fungi.

3. The strain of *Bacillus halotolerans* of claim 1, wherein the plant pathogenic fungi comprise one or more of *Lasiodiplodia theobromae*, *Botryosphaeria dothidea*, *Neofusicoccum parvum, Diaporthe sojae*, *Diaporthe eres, Diaporthe honkonggensis*, *Botrytis cinerea*, *Colletotrichum viniferum*, *Colletotrichum aenigma*, *Colletotrichum gloeosporioides*, *Colletotrichum fructicola*, *Colletotrichum siamense*, *Colletotrichum acutatum*, *Neopestalotiopsis sp.*, *Neopestalotiopsis rosae*, *Coniella vitis*, *Dactylonectria macrodidyma*, *Fusarium oxysporum*, anastomosis group A of *binucleate Rhizoctonia*, anastomosis group G of *binucleate Rhizoctonia*, *Alternaria alternata*, and *Sclerotinia sclerotiorum.*

4. Use of the strain of *Bacillus halotolerans* of any one of claims 1 to 3 in preparing a biocontrol agent or a microbial fertilizer for inhibiting plant pathogenic fungi.

5. The use of claim 4, wherein the plant pathogenic fungi comprise one or more of *Lasiodiplodia theobromae*, *Botryosphaeria dothidea*, *Neofusicoccum parvum*, *Diaporthe sojae*, *Diaporthe eres*, *Diaporthe honkonggensis*, *Botrytis cinerea*, *Colletotrichum viniferum*, *Colletotrichum aenigma*, *Colletotrichum gloeosporioides*, *Colletotrichum fructicola*, *Colletotrichum siamense*, *Colletotrichum acutatum*, *Neopestalotiopsis sp.*, *Neopestalotiopsis rosae*, *Coniella vitis*, *Dactylonectria macrodidyma*, *Fusarium oxysporum*, anastomosis group A of *binucleate Rhizoctonia*, anastomosis group G of *binucleate Rhizoctonia*, *Alternaria alternata*, and *Sclerotinia sclerotiorum.*

6. A biocontrol agent against plant pathogenic fungi, wherein an active ingredient of the biocontrol agent is the strain of *Bacillus halotolerans* of any one of claims 1 to 3.

7. The biocontrol agent of claim 6, wherein the plant pathogenic fungi comprise one or more of *Lasiodiplodia theobro-*

*mae*, *Botryosphaeria dothidea*, *Neofusicoccum parvum*, *Diaporthe sojae*, *Diaporthe eres*, *Diaporthe honkonggensis*, *Botrytis cinerea*, *Colletotrichum viniferum*, *Colletotrichum aenigma*, *Colletotrichum gloeosporioides*, *Colletotrichum fructicola*, *Colletotrichum siamense*, *Colletotrichum acutatum*, *Neopestalotiopsis sp.*, *Neopestalotiopsis rosae*, *Coniella vitis*, *Dactylonectria macrodidyma*, *Fusarium oxysporum*, anastomosis group A of *binucleate Rhizoctonia*, anastomosis group G of *binucleate Rhizoctonia*, *Alternaria alternata*, and *Sclerotinia sclerotiorum.*

**8.** A microbial fertilizer against plant pathogenic fungi, wherein an active ingredient of the biocontrol agent is the strain of *Bacillus halotolerans* of any one of claims 1 to 3.

**9.** The microbial fertilizer of claim 8, wherein the plant pathogenic fungi that cause plant diseases comprise one or more of *Lasiodiplodia theobromae*, *Botryosphaeria dothidea*, *Neofusicoccum parvum*, *Diaporthe sojae*, *Diaporthe eres*, *Diaporthe honkonggensis*, *Botrytis cinerea*, *Colletotrichum viniferum*, *Colletotrichum aenigma*, *Colletotrichum gloeosporioides*, *Colletotrichum fructicola*, *Colletotrichum siamense*, *Colletotrichum acutatum*, *Neopestalotiopsis sp.*, *Neopestalotiopsis rosae*, *Coniella vitis*, *Dactylonectria macrodidyma*, *Fusarium oxysporum*, anastomosis group A of *binucleate Rhizoctonia*, anastomosis group G of *binucleate Rhizoctonia*, *Alternaria alternata*, and *Sclerotinia sclerotiorum.*

**10.** Use of the strain of *Bacillus halotolerans* of any one of claims 1 to 3 in promoting growth of grape seedlings.

**11.** A method for promoting growth of grape seedlings, comprising irrigating roots of the grape seedlings using a fermentation medium, a diluent of the fermentation medium, a fermentation medium filtrate or a diluent of fermentation medium filtrate of the strain of *Bacillus halotolerans* of any one of claims 1 to 3.

**12.** The method of claim 11, comprising irrigating, when the grape seedlings grow to have 5-7 leaves, the roots of the grape seedlings with the diluent of the fermentation medium filtrate of the strain of *Bacillus halotolerans* of any one of claims 1 to 3.

FIG. 1

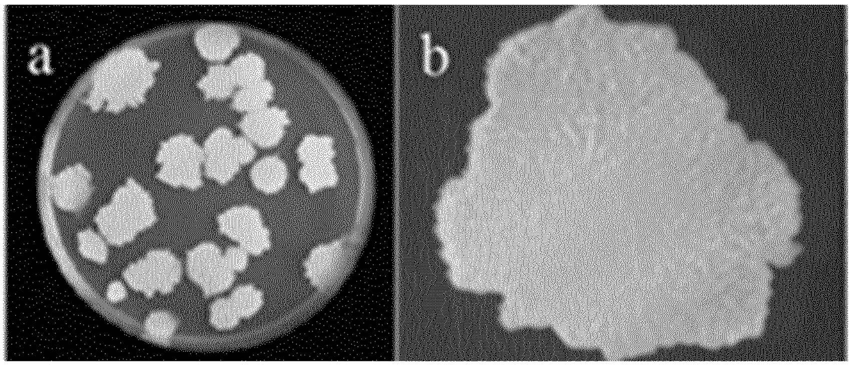


FIG. 2

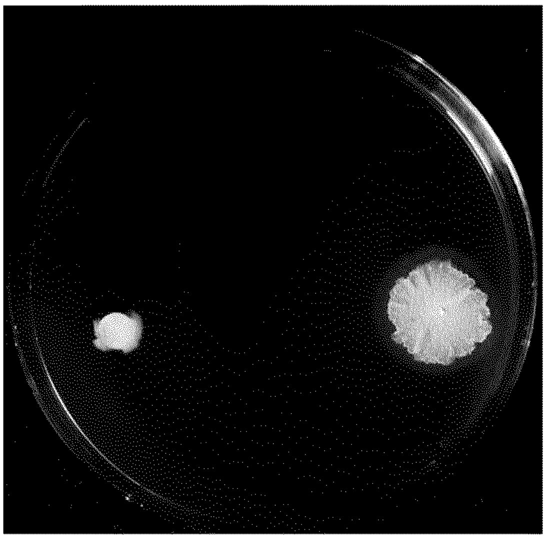

FIG. 3

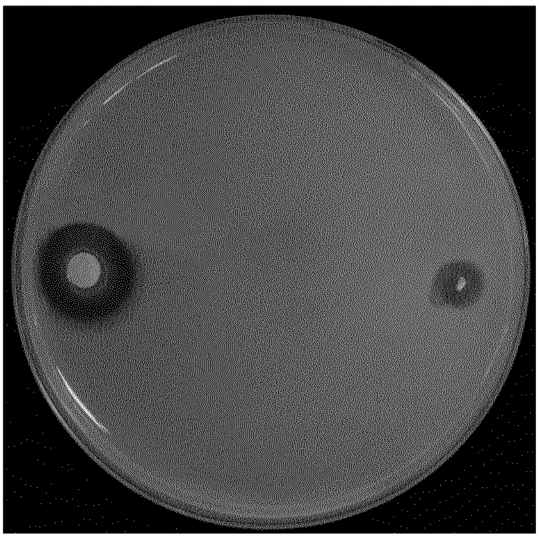

FIG. 4

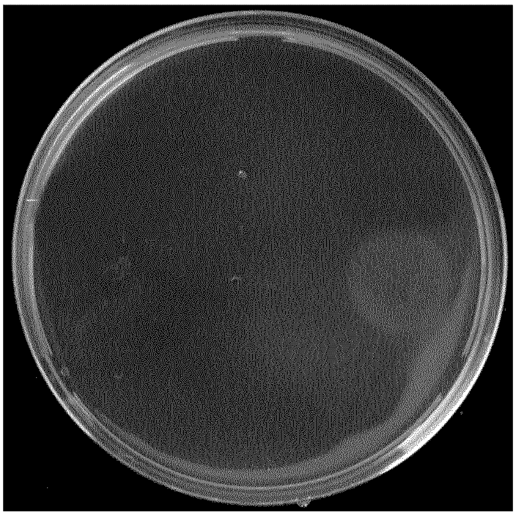

FIG. 5

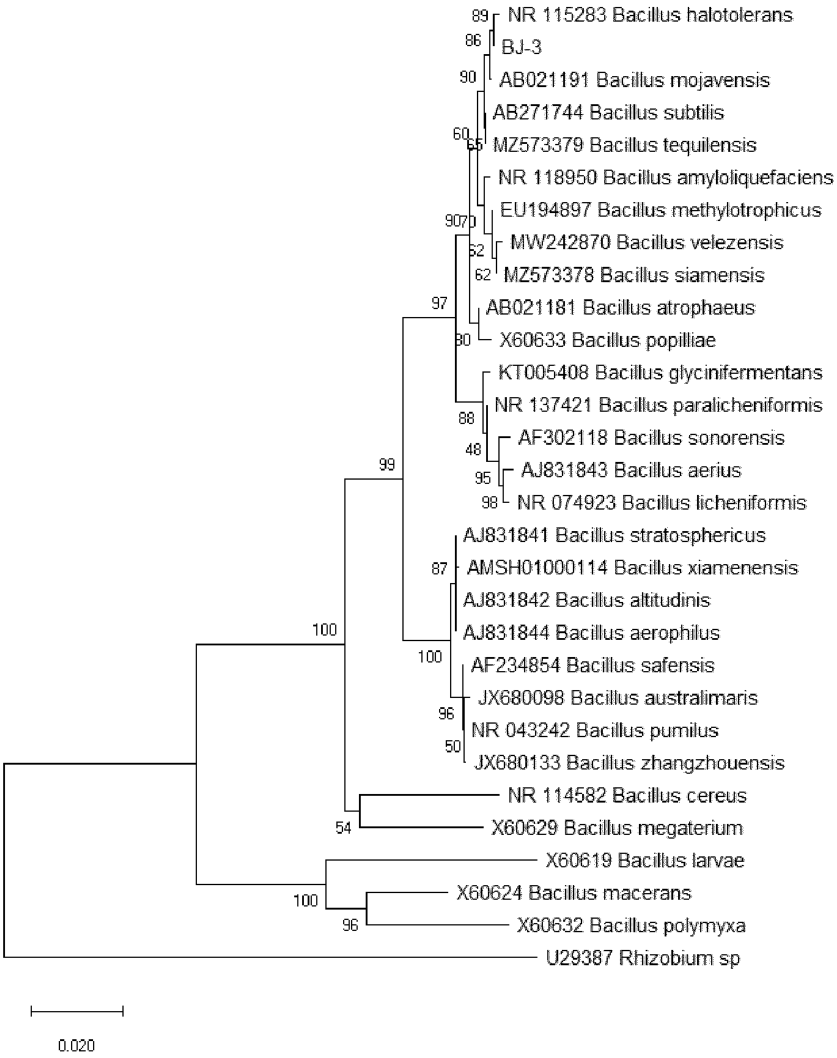
NR 115283 Bacillus halotolerans
BJ-3
AB021191 Bacillus mojavensis
AB271744 Bacillus subtilis
MZ573379 Bacillus tequilensis
NR 118950 Bacillus amyloliquefaciens
EU194897 Bacillus methylotrophicus
MW242870 Bacillus velezensis
MZ573378 Bacillus siamensis
AB021181 Bacillus atrophaeus
X60633 Bacillus popilliae
KT005408 Bacillus glycinifermentans
NR 137421 Bacillus paralicheniformis
AF302118 Bacillus sonorensis
AJ831843 Bacillus aerius
NR 074923 Bacillus licheniformis
AJ831841 Bacillus stratosphericus
AMSH01000114 Bacillus xiamenensis
AJ831842 Bacillus altitudinis
AJ831844 Bacillus aerophilus
AF234854 Bacillus safensis
JX680098 Bacillus australimaris
NR 043242 Bacillus pumilus
JX680133 Bacillus zhangzhouensis
NR 114582 Bacillus cereus
X60629 Bacillus megaterium
X60619 Bacillus larvae
X60624 Bacillus macerans
X60632 Bacillus polymyxa
U29387 Rhizobium sp
0.020

FIG. 6

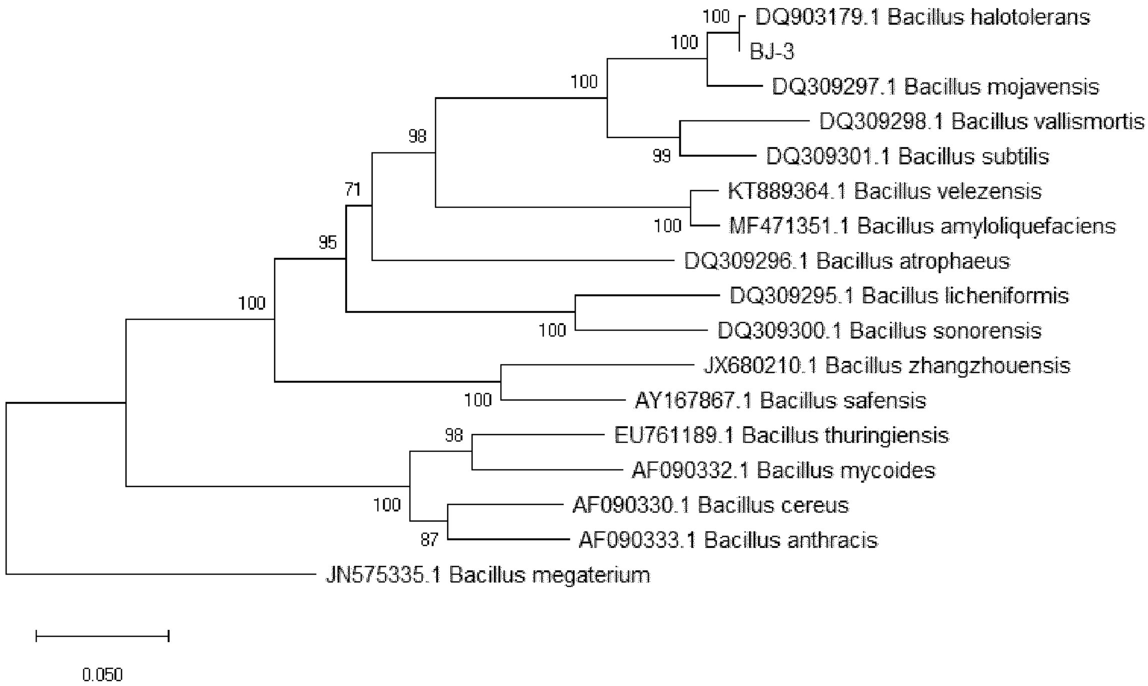
100
DQ903179.1 Bacillus halotolerans
100
BJ-3
100
DQ309297.1 Bacillus mojavensis
98
DQ309298.1 Bacillus vallismortis
99
DQ309301.1 Bacillus subtilis
71
KT889364.1 Bacillus velezensis
100
MF471351.1 Bacillus amyloliquefaciens
95
DQ309296.1 Bacillus atrophaeus
100
DQ309295.1 Bacillus licheniformis
100
DQ309300.1 Bacillus sonorensis
JX680210.1 Bacillus zhangzhouensis
100
AY167867.1 Bacillus safensis
98
EU761189.1 Bacillus thuringiensis
AF090332.1 Bacillus mycoides
100
AF090330.1 Bacillus cereus
87
AF090333.1 Bacillus anthracis
JN575335.1 Bacillus megaterium
0.050

FIG. 7

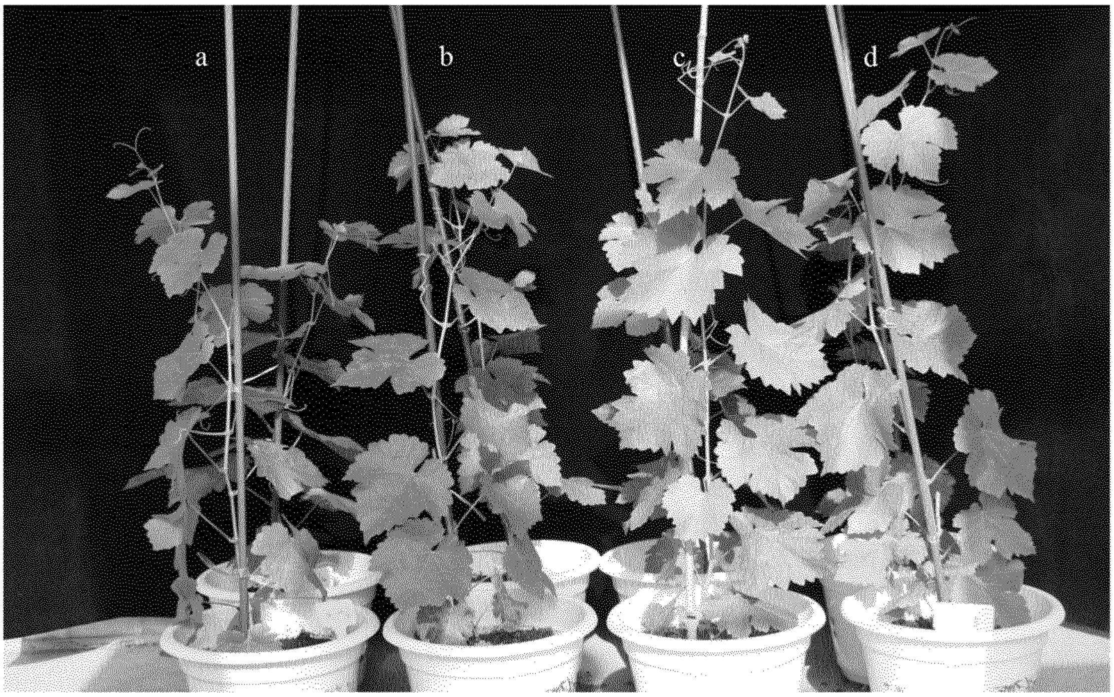
a
b
c
d

FIG. 8

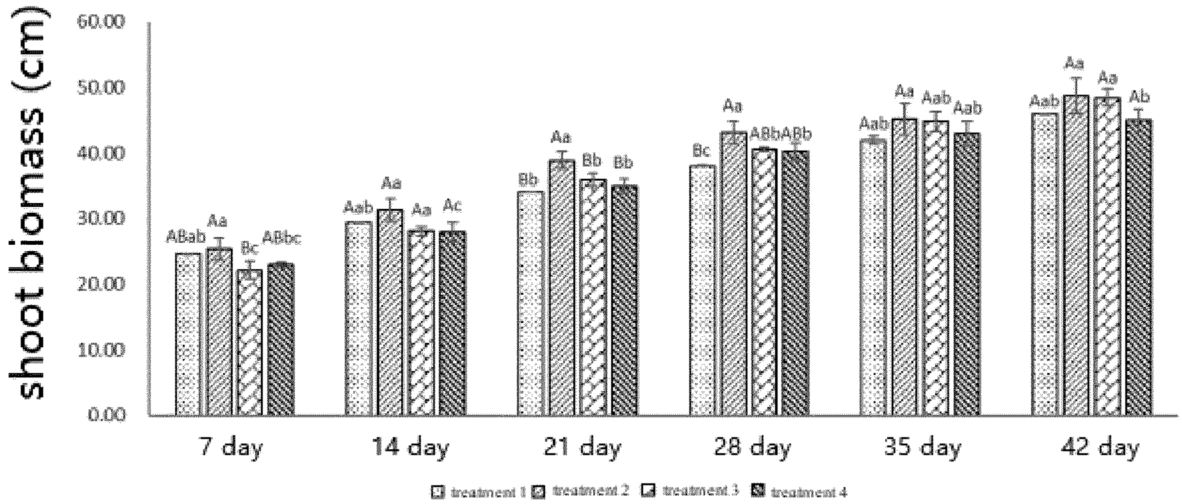
shoot biomass (cm)
60.00
50.00
40.00
30.00
20.00
10.00
0.00
ABab
Aa
Bc
ABbc
Aab
Aa
Aa
Ac
Bb
Aa
Bb
Bb
Bc
Aa
ABb
ABb
Aab
Aa
Aab
Aab
Aab
Aa
Aa
Ab
7 day
14 day
21 day
28 day
35 day
42 day
treatment 1
treatment 2
treatment 3
treatment 4

FIG. 9

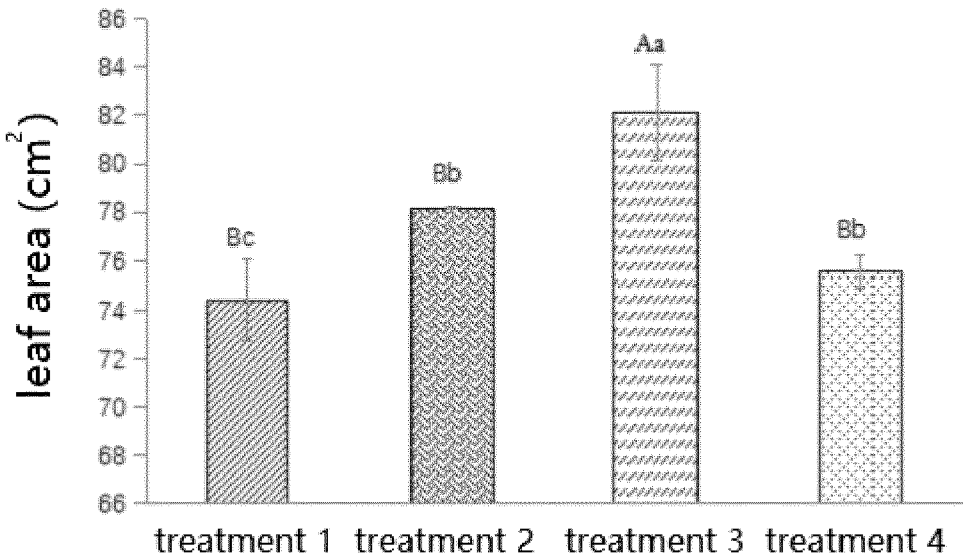
leaf area ($cm^2$)
86
84
82
80
78
76
74
72
70
68
66
Bc
Bb
Aa
Bb
treatment 1
treatment 2
treatment 3
treatment 4

FIG. 10

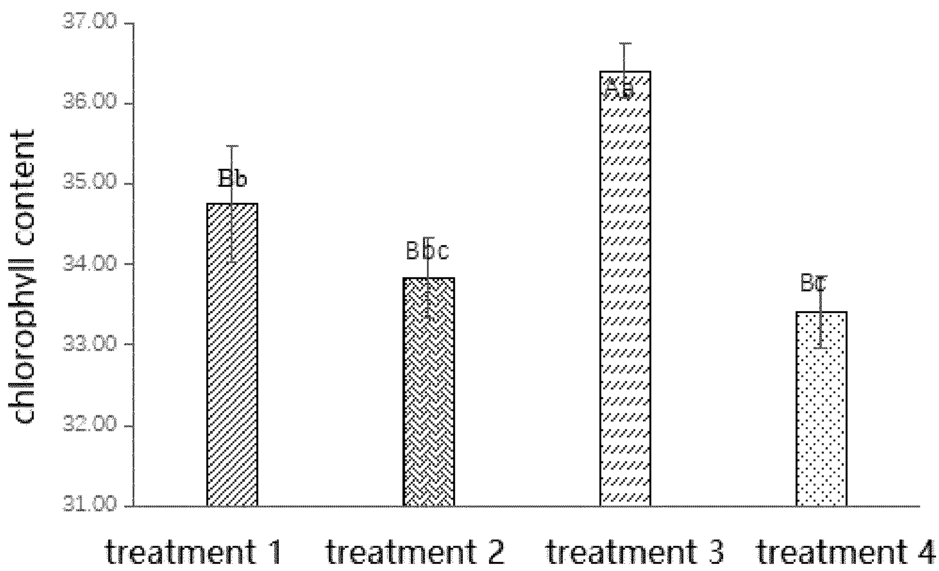
chlorophyll content
37.00
36.00
35.00
34.00
33.00
32.00
31.00
Bb
Bbc
Aa
Bc
treatment 1
treatment 2
treatment 3
treatment 4

**INTERNATIONAL SEARCH REPORT**

International application No.
**PCT/CN2022/130479**

**A. CLASSIFICATION OF SUBJECT MATTER**

C12N 1/20(2006.01)i;A01G 17/02(2006.01)i;A01G 13/00(2006.01)i;A01N 63/22(2020.01)i;A01P 3/00(2006.01)i;A01P 21/00(2006.01)i;C05F 11/08(2006.01)i;C12R 1/07(2006.01)n

According to International Patent Classification (IPC) or to both national classification and IPC

**B. FIELDS SEARCHED**

Minimum documentation searched (classification system followed by classification symbols)

IPC: C12N, A01G, A01N, A01P, C05F, C12R

Documentation searched other than minimum documentation to the extent that such documents are included in the fields searched

Electronic data base consulted during the international search (name of data base and, where practicable, search terms used)

CPRSABS, CNABS, CNTXT, USTXT, WOTXT, EPTXT, CJFD, CSCD, SIPONPL, DWPI, SIPOABS, CPEA, CNKI, ISI WEB OF SCIENCE, MEDLINE, PUBMED, 万方数据库, WANFANG: 耐盐芽孢杆菌, 芽孢杆菌, BJ-3, CGMCC 3w 24115, 植物病原真菌, 植物病原菌, Bacillus halotolerans, Bacillus, plant pathogenic fungi, plant pathogen.

**C. DOCUMENTS CONSIDERED TO BE RELEVANT**

| Category* | Citation of document, with indication, where appropriate, of the relevant passages | Relevant to claim No. |
|---|---|---|
| PX | CN 115058358 A (BEIJING ACADEMY OF AGRICULTURE AND FORESTRY SCIENCES) 16 September 2022 (2022-09-16)<br>see claims 1-12 | 1-12 |
| A | CN 111979149 A (INSTITUTE OF PLANT PROTECTION, GANSU ACADEMY OF AGRICULTURAL SCIENCES) 24 November 2020 (2020-11-24)<br>see claims 1-9, and embodiments 1-4 | 1-12 |
| A | CN 114231444 A (PLANT PROTECTION RESEARCH INSTITUTE OF HENBEI ACADEMY OF AGRICULTURE AND FORESTRY) 25 March 2022 (2022-03-25)<br>see claims 1-10, and embodiments 1-4 | 1-12 |
| A | JP 2005151887 A (IDEMITSU KOSAN CO.) 16 June 2005 (2005-06-16)<br>see claims 1-9, and embodiments | 1-12 |
| A | CN 101659932 A (NANJING AGRICULTURAL UNIVERSITY) 03 March 2010 (2010-03-03)<br>see claims 1-7, and specific embodiments | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 March 2023** | **13 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/130479**

| C. DOCUMENTS CONSIDERED TO BE RELEVANT | | |
|---|---|---|
| Category* | Citation of document, with indication, where appropriate, of the relevant passages | Relevant to claim No. |
| A | 张帅等 (ZHANG, Shuai et al.). "一株具有抗菌活性耐盐芽孢杆菌B-11的分离鉴定及发酵工艺优化 (Identification of Bacillus sp. B-11 and its Optimization of Fermentation Conditions for Antimicrobial Activity Against Staphylococcus Aureus)" *中国抗生素杂志 (Chinese Journal of Antibiotics),* Vol. 41, No. 7, 31 July 2016 (2016-07-31), ISSN: 1001-8689, see abstract | 1-12 |
| A | WANG, Fang et al. "Biocontrol ability and action mechanism of Bacillus halotolerans against Botrytis cinerea causing grey mould in postharvest strawberry fruit" *Postharvest BiologyandTechnology,* Vol. 174, 07 January 2021 (2021-01-07), ISSN: 0925-5214, see Abstract | 1-12 |

Form PCT/ISA/210 (second sheet) (July 2022)

**INTERNATIONAL SEARCH REPORT**

International application No.
**PCT/CN2022/130479**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2022/130479**

| Patent document cited in search report | | | Publication date (day/month/year) | Patent family member(s) | | | Publication date (day/month/year) |
|---|---|---|---|---|---|---|---|
| CN | 115058358 | A | 16 September 2022 | None | | | |
| CN | 111979149 | A | 24 November 2020 | None | | | |
| CN | 114231444 | A | 25 March 2022 | None | | | |
| JP | 2005151887 | A | 16 June 2005 | None | | | |
| CN | 101659932 | A | 03 March 2010 | EP | 2489725 | A1 | 22 August 2012 |
| | | | | EP | 2489725 | A4 | 20 November 2013 |
| | | | | KR | 20110034578 | A | 05 April 2011 |
| | | | | KR | 101039176 | B1 | 03 June 2011 |
| | | | | US | 2012045427 | A1 | 23 February 2012 |
| | | | | US | 8476057 | B2 | 02 July 2013 |
| | | | | EP | 3613843 | A1 | 26 February 2020 |
| | | | | EP | 3613843 | B1 | 09 June 2021 |
| | | | | AU | 2009292605 | A1 | 02 June 2011 |
| | | | | AU | 2009292605 | B2 | 12 September 2013 |
| | | | | WO | 2011032330 | A1 | 24 March 2011 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **QINGTONG YE ; YAMENG LI ; YUEYAN ZHOU et al.** Occurrence of grapevine trunk diseases caused by fungal pathogens in the domestic and overseas. *Journal of Fruit Science,* 2021, vol. 38 (2), 278-292
- **HOFSTETTER V ; BUYCK B ; CROLL D et al.** What if esca disease of grapevine were not a fungal disease?. *Fungal Diversity,* 2012, vol. 54 (1), 51-67
- **YAN J Y ; XIE Y ; ZHANG W et al.** Species of Botryosphaeriaceae involved in grapevine dieback in China. *Fungal Diversity.,* 2013, vol. 61, 221-236
- **MANAWASINGHE I S ; DISSANAYAKE A J ; LI X et al.** High genetic diversity and species complexity of Diaporthe associated with grapevine dieback in China. *Frontiers in Microbiology,* 2019, vol. 10, 1936
- **RUSIN C ; ROSSICAVALCANTI F ; LIMA P et al.** Control of the fungi Lasiodiplodia theobromae, the causal agent of dieback, in cv. syrah grapevines. *Acta Scientiarum Agronomy,* 2020, vol. 43, e44785
- **EIRINI-EVANGELIA THOMLOUDI ; PANAGIOTIS KATINAKIS.** Genomic and metabolomic insights into secondary metabolites of the novel Bacillus halotolerans Hil4, an endophyte with promising antagonistic activity against gray mold and plant growth promoting potential. *Microorganisms,* 2021, vol. 9, 2508
- **MUHAMMAD NADEEM HASSAN.** *Vegetable associated Bacillus*
- **FUSARIUM SOLANI ; RAHEELA RIAZ.** *Biological Control,* 2021, vol. 1, 104610
- Strain of Bacillus halotolerans LYSX1-induced systemic resistance against the root-knot nematode Meloidogyne javanica in tomato. *Ann Microbiol,* 2019, vol. 69, 1227-1233
- **HUAILAI COUNTY ; ZHANGJIAKOU, HEBEI PROVINCE.** isolated from the trunk tissues with grapevine trunk diseases, and named as Bacillus halotolerans BJ-3,which was deposited. *General Microbiology Center of China Committee of Microbiological Culture Collection and Management (Address: No. 3, No. 1 Courtyard, West Beichen Road, Chaoyang District, Beijing),* 16 December 2021
- **COURTYARD, WEST BEICHEN ROAD ; CHAOYANG DISTRICT, BEIJING.** *Institute of Microbiology, Chinese Academy of Sciences,* 16 December 2021, (3

## Sequence Listing

<110> Beijing Academy of Agriculture and Forestry Sciences

<120> A strain of *Bacillus halotolerans* and application thereof

<130> WHOI220034

<160> 2

<170> PatentIn version 3.5

<210> 1

<211> 1446

<212> DNA

<213> *Bacillus halotolerans*

<400> 1

ctagcgctgc tatactgcaa gtcgagcgga cagatgggag cttgctccct gatgttagcg 60
gcggacgggt gagtaacacg tgggtaacct gcctgtaaga ctgggataac tccgggaaac 120
cggggctaat accggatgct tgtttgaacc gcatggttca aacataaaag gtggcttcgg 180
ctaccactta cagatggacc cgcggcgcat tagctagttg gtgaggtaat ggctcaccaa 240
ggcaacgatg cgtagccgac ctgagagggt gatcggccac actgggactg agacacggcc 300
cagactccta cgggaggcag cagtagggaa tcttccgcaa tggacgaaag tctgacggag 360
caacgccgcg tgagtgatga aggttttcgg atcgtaaagc tctgttgtta gggaagaaca 420
agtaccgttc gaatagggcg gtaccttgac ggtacctaac cagaaagcca cggctaacta 480
cgtgccagca gccgcggtaa tacgtaggtg gcaagcgttg tccggaatta ttgggskwaa 540
agggytcsca ggcggttcct taagtytgaw gkgaaagccc ccggytcaac cggggagggt 600
cattggaaac tggggaactt gagtgcagaa gaggagagtg gaattccacg tgtagcggtg 660
aaatgcgtag agatgtggag gaacaccagt ggcgaaggcg actctctggt ctgtaactga 720
cgctgaggag cgaaagcgtg gggagcgaac aggattagat accctggtag tccacgccgt 780
aaacgatgag tgctaagtgt tagggggttt ccgcccctta gtgctgcagc taacgcatta 840
agcactccgc ctggggarta cggtcgcaag actgaaactc maaggaattg acgggggccc 900
gcacaagcgg kgragcwtgk ggtttawtyc raagcamsgc gaagaacctt accaggtctt 960
gacatcctct gacaatccta gagataggac gtccccttcg ggggcagagt gacaggtggt 1020
gcatggttgt cgtcagctcg tgtcgtgaga tgttgggtta agtcccgcaa cgagcgcaac 1080
ccttgatctt agttgccagc attcagttgg gcactctaag gtgactgccg gtgacaaacc 1140
ggaggaaggt ggggatgacg tcaaatcatc atgcccctta tgacctgggc tacacacgtg 1200
ctacaatgga cagaacaaag ggcagcgaaa ccgcgaggtt aagccaatcc cacaaatctg 1260
ttctcagttc ggatcgcagt ctgcaactcg actgcgtgaa gctggaatcg ctagtaatcg 1320
cggatcagca tgccgcggtg aatacgttcc cgggccttgt acacaccgcc cgtcacacca 1380
cgagagtttg taacacccga agtcggtgag gtaaccttta tgagccagcc gccgaaggga 1440

cccccct 1446

<210> 2

<211> 872

<212> DNA

<213> *Bacillus halotolerans*

<400> 2
ggtcgttacg cgttatcaac agagcttgat gtgactgttc accgtgacgg aaaaatccat 60
cgccaagtct ataaccgcgg tgtcccggtt tctgatcttg aggttattgg cgaaacggat 120
catacaggaa cgactacaca ctttgttcca gatcctgaaa tcttcacgga aacaactgag 180
tatgaatatg atttgcttgc taaccgtgtt cgcgaactag cctttttgac aaaaggcgta 240
aacatcacga ttgaagataa acgtgaaggc caagagcgca aaaatgagta tcattacgaa 300
ggcggaatta aaagctatgt agagtattta aaccgctcca aagaagttgt ccatgaagag 360
ccgatttata ttgaaggcga aaaggacggc attacggttg aagtcgctct gcaatacaat 420
gacagctaca caagcaatat ttactcattt acaaacaata tcaacacgta cgaaggcggt 480
acccacgaag ccggttttaa aacggggctg actcgtgtca tcaatgatta cgccagaaaa 540
aaaggactca taaaagaaaa tgatccaaac ttaagcggag atgatgtgag agaagggctt 600
accgcgatta tctcgatcaa acacccggat ccgcagttcg aaggccaaac gaaaacaaaa 660
ctaggcaact cagaggcgcg gacgatcaca gatacgttat tttctgctgc gttggaaacc 720
tttatgctgg aaaatccaga tgcggccaaa aaaatcgttg acaaaggctt aatggcagca 780
agagcaagaa tggctgcgaa aaaagcgcgt gaattaacgc gccgcaaaag cgctttggag 840
atttcaaacc ttcctggtaa attagcggac tg 872